# EUROPEAN PATENT SPECIFICATION

(11) **EP 2 831 333 B1**
(45) Date of publication and mention of the grant of the patent: **02.07.2025**
(21) Application number: 13723572.7
(22) Date of filing: 27.03.2013
(51) Int. Cl.: D06M 11/00, A61Q 19/00, A61L 27/60, D06M 16/00, D06M 13/00

(54) **METHOD FOR TREATMENT OF SPIDER SILK-FILAMENT FOR USE AS THREAD OR A COMPOSITION IN THE MANUFACTURE OF COSMETIC, MEDICAL, TEXTILE OR INDUSTRIAL APPLICATIONS SUCH AS BIO-ARTIFICIAL CELL TISSUE OR ARTIFICIAL SKIN BASED ON SPIDER SILK DERIVED FROM GENETICALLY MODIFIED ORGANISMS**
VERFAHREN ZUR BEHANDLUNG VON SPINNENSEIDENFILAMENT ZUR VERWENDUNG ALS GARN ODER ZUSAMMENSETZUNG BEI DER HERSTELLUNG KOSMETISCHER, MEDIZINISCHER, TEXTIL- ODER INDUSTRIELLER ANWENDUNGEN WIE ETWA BIOLOGISCH-KÜNSTLICHES ZELLGEWEBE ODER KÜNSTLICHE HAUT AUF BASIS VON SPINNENSEIDE AUS GENETISCH VERÄNDERTEN ORGANISMEN
PROCÉDÉ POUR LE TRAITEMENT DE FILAMENT DE SOIE D'ARAIGNÉE POUR UTILISER COMME FIL OU COMPOSITION DANS LA FABRICATION DE PRODUITS COSMÉTIQUES, MÉDICAUX, TEXTILES OU DANS DES APPLICATIONS INDUSTRIELLES COMME DES TISSUS DE CELLULES BIO-ARTIFICIELLES OU DE LA PEAU ARTIFICIELLE À BASE DE SOIE D'ARAIGNÉE (RECOMBINÉE)

(30) Priority: 27.03.2012 NL 1039495
(43) Date of publication of application: 04.02.2015
(73) Proprietor: Essaidi, Jalila, 5502 HV Veldhoven (NL)
(72) Inventor: Essaidi, Jalila, 5502 HV Veldhoven (NL)
(74) Representative: Algemeen Octrooi- en Merkenbureau B.V.
(86) International application number: PCT/NL2013/000018
(87) International publication number: WO 2013/147590

(56) References cited:
- WO-A1-97/08315
- WO-A2-2004/090205
- WO-A2-2006/017701
- WO-A2-2011/008842
- ANONYMOUS: "alum | chemical compound | Britannica.com", 12 April 2007 (2007-04-12), XP055299759, Retrieved from the Internet <URL:https://www.britannica.com/science/alum> [retrieved on 20160905]
- SCHELLER JUERGEN ET AL: "Production of spider silk proteins in tobacco and potato", NATURE BIOTECHNOLOGY, NATURE PUBLISHING GROUP, NEW YORK, NY, US, vol. 19, no. 6, 1 June 2001 (2001-06-01), pages 573 - 577, XP002189903, ISSN: 1087-0156, DOI: 10.1038/89335

## Description

Artist and inventor Jalila Essaidi of the above new methods, new textile fabric, new support structure and new stronger, growing living skin is a practitioner of the emerging art movement "BioArt" which uses living biological material as a medium.

The source material for this invention is derived from biotechnology. The inventor has studied the use of spider silk and silk when applied to tissues. The word tissue used in this method refers to tissues in the broadest sense: a surface, organic and biological in nature such as (artificial) cell tissues or skin tissue (artificial skin) for applications for the treatment of burns and open wounds; but also textile woven fabric made of threads or yarns; as well as surfaces of objects such as socks and cars.

For her bullet resistant art-project Essaidi was the first in the world to mechanically weave recombinant spider silk from genetically modified silkworms. Based on this material she created a stronger, protective artificial (human) skin for medical and cosmetic applications. For this invention with: 'a stronger, protective skin for medical and cosmetic applications', we mean an inventively obtained skin substitute or artificial skin based on spider silk, as well as a skin treated with a spider silk-based skin cream. Studies on the properties of silk indicate that natural spider silk has a high potential for thermal conductivity and when used in biological applications a faster wound healing can occur, less scar tissue is formed and the regeneration process of skin cells is stimulated. A conceptual application of spider silk seemed for example bulletproof vests because spider silk is many times stronger, more elastic and more moisture and temperature resistant than Kevlar. Also, the application as a suture for medical purposes is well known. The inventor, inspired by these known conceptual applications, considered that when spider silk can be successfully used as suture, then it is likely there will occur neither an immune response when new and inventively combined with tissue, nor an allergic reaction when new and inventively combined with skin cream. Also, the inventor considered that when natural spider silk has a high potential for thermal conductivity, then it will be plausible that a textile fabric and / or supporting structure based on spider silk filament and other two-and three-dimensional inventively obtained constructs based on spider silk from genetically modified organisms, also have high thermal conductive characteristics.

### BACKGROUND OF THE INVENTION

Spider silk is a protein fiber spun by many different spiders. It has a variety of functions: the protection of eggs, the use as dragline, and the use as material for a web that can withstand high impacts and to catch insects. Spider silk comes in different types; for example, an orb-weaver spider silk can produce up to six different types, each with different mechanical properties.

### 1. Important properties of spider silk

Spider silk is stronger in terms of energy to break than Kevlar (up to 5 fold), but also more elastic than Nylon. It has a tensile strength of up to six times the strength of steel. Moreover, spider silk is also stable over a wide temperature up to 250°C, its thermal conductivity is very high, it is flexible and insoluble in many organic and aqueous solvents and weak acids and bases, nevertheless it is slowly biodegradable.

A scaffold for human skin equivalents should break down at a similar pace as the growth of new tissue, so that the new tissue can be integrated into the surrounding host tissue. This degradation process in both silk and spider silk is a result of proteolysis, with protease being reported to have the greatest effect.

The main feature of spider silk for use in artificial skin and human skin equivalents is bio-compatibility. Normal silk has been used for suture material since the end of the 19th century on a commercial scale and has proven to be an effective biomaterial, however, it is not fully bio-compatible. Spider silks (spidroin) unlike normal silks (fibroin) do not have an immunogenic sericin coating, which in combination with fibroin is identified as the source of these immunogenic reactions. Numerous studies have demonstrated that once sericin is extracted from the normal silk, cell adhesion, proliferation, growth and function of a variety of cell types increases. However, when the sericin is extracted from the normal silk it results in changes of the mechanical properties of this silk. Which characteristics and the extent of the changes are dependent on the method used. The fact that spider silk does not have this immunogenic sericin coating and thus is bio-compatible without additional processing makes it the ideal choice for a scaffold for human skin equivalents.

The main feature of spider silk for use in artificial constructs is the heat transfer properties of spider silk described by Huang, X., Liu, H. Wang, X., New Secrets of Spider Silk: Exceptionally High Thermal Conductivity and Its Abnormal Change under Stretching. Advanced Materials 2012. Which demonstrated that the thermal conductivity of spider silk is better than most materials including silicon, aluminum and pure iron.

### 2. Different production methods of spider silk

In order to obtain spider silk (filaments) in a natural way, spiders have to be kept separated in large areas and intensively cared for. Partly because of this, the harvesting of large quantities of spider silk (for industrial purposes) is not feasible and some parties are working on alternative strategies to produce large quantities of spider silk. Worldwide transgenic modifications of various organisms, including goats, bacteria, alfalfa and silkworms are being researched. In these organisms the gene is expressed responsible for the production of a spider silk protein, with the aim that this new organism itself can produce this spider silk protein.

In most of these studies, however, the harvesting of large quantities of spider silk is far from commercially interesting due to the high production cost. Recent research into silkworms offers here a promising future, several studies conducted by both Hongxiu Wen and Randy Lewis resulted in the creation of a transgenic silkworm that produces spider silk proteins: the chemical and physical properties of spider silk combined with the domesticated silkworm, selected for ease of use. Described in Teulé F, Miao YG, Sohn BH, Kim YS, Hull JJ, Fraser MJ Jr, Lewis RV, Jarvis DL., Silkworms transformed with chimeric silkworm/spider silk genes spin composite silk fibers with improved mechanical properties. PNAS U S A. 2012 January 17; 109(3): 923-928.

The spider silk used for the bullet resistant art-project resulted from research on the basis of the NIH Grant, which was granted to Randy Lewis, Don Jarvis and Mac Frasier. With the help of this Grant several different types of genetically modified silkworms were created that produced spider silk in their silk. A special silkworm has also been made: a platform-worm ready for genetic modification aimed at tailor-made spider silk with the desired mechanical properties, tailored to the needs of the researchers.

### 3. Scaffolds for tissue engineering based on spider silk

The use of silks for scaffolds in tissue engineering is widely researched. The various types of scaffolds based on traditional silk, described below, could also be made using spider silk. Changes of the mechanical properties of silk caused by the removal of sericin are no longer of importance when silk is dissolved. In this most simple form silk can be processed as a film or a coating for other materials, it may be treated by salt leaching in order to create a porous scaffold, and a spongy or porous scaffold can be obtained by freeze drying, gas foaming and phase separation. Silk solutions can also be turned into fibers with the aid of electro-spinning, which can be twined into a thread to be used in scaffolds. Dissolved silk can even be used as a base ingredient for hydrogels, which may function as a scaffold.

For the development of the scaffold used for the bullet resistant art-project no dissolved spider silk had been used, instead a recombinant spider silk thread (filament) was used, coming directly from a cocoon of a genetically modified silkworm. The spider silk was obtained from Randy Lewis on a cone having thereon the unwound cocoon. In order to be able to weave this material three strands of spider silk filament had to be twined to form one thread.

### 4. Different skin models

In recent years human skin equivalents (artificial skin) have become increasingly important in various fields. Reconstructed epidermis models already replace all animal tests for skin corrosion and irritation. They are used for testing cosmetics according to cosmetic regulations. More complex skin models are used as research tools for the cosmetic and pharmaceutical industry as well as academia. Especially in this field the applications and diversity of skin models is growing quickly.

### 5. Human skin equivalents (artificial skin) based on spider silk

The aim of the bullet resistant art-project of the inventor was to create an human skin equivalent model that was as close as possible to human skin, this in order to get the project truly "under your skin". Because of this the inventor choose for a full thickness human skin equivalent based on human skin cells isolated from cosmetic procedures such as breast reductions, abdominoplasty and circumcisions.

The emphasis in the above-description of the background of the invention is on the use of the invention as a human skin equivalent. However, this is only one example of the possible applications for which the invention lends itself.

WO 2004/090205 A2 discloses treatment of spider silk filaments with agents such as antimicrobials, which are preserving agents and medical/dermatological active substances.

WO 97/08315 A1 discloses treatment of spider silk filaments with dyes or pigments.

WO 2006/017701 A2 discloses coating spider silk filaments with modifiers such as antibiotics, hormones, biocides, dyes, pigments.

### SUMMARY OF THE INVENTION

By inventively treating, twining, braiding or cabling one, two or more (recombinant) spider silk filaments derived from genetically modified organisms, or obtained through a wet or electro-spinning process of spider silk protein derived from genetically modified organisms, a spider silk thread is created which among other things is stronger than its alternatives and optimized for its application.

The present invention relates to a method for manufacturing a spider silk thread on basis of a spider silk filament in the manufacture of cosmetic, medical, textile or industrial applications as recited in the wording of claim 1.

Preferably, the spider silk filament is obtained by a wet or electro-spinning process of spider silk protein derived from genetically modified organisms.

The manufacture of a textile fabric and/or support structure, results in a material much stronger and more flexible than the present-day alternatives, based on the above-described spider silk thread.

The manufacture of a textile fabric and/or support structure, based on the above-described spider silk thread, comprising an innovative enlarged total surface area, which is suitable for use in heat exchange with a medium such as solids, liquids and gases, as well as for the application as a support structure for liquids which harden or organic materials which grow together.

The above-described textile fabrics may be used as a cheaper and more flexible heat conductor for a wide range of applications. The obtained total surface can be tailored to its target, and the application can be (partly) thermal insulated to reduce heat loss to a minimum.

The above-described textile fabrics and/or support structure may also be used as a scaffold for tissue engineering.

The above-described scaffold shows, because of the use of spider silk filament and the applied weave, a better cell-adhesion when applied for tissue engineering.

The above-described scaffold stimulates, through its material, weave and treatment, the growth and function of cells to an increased degree, and has significant influence on skin growth.

The above-described scaffold degrades in the human body, through its material and weave, with a controlled speed.

The above-described scaffold shows, because of its material, weave, treatment and the speed at which it degrades in the body, a reduced pathological immune response.

Through the application of the above-described scaffold for tissue engineering, a skin is created which exhibits a prolonged growth and stimulates stem cell production. Through the application of the above-described scaffold for tissue engineering, a stronger, reinforced artificial tissue or skin tissue (bullet resistant tissue) is obtained with a number of new features, for example, enhanced tolerance for impacts from bullets and shrapnel.

All of the above-described treatments and applications apply also for similar non-woven constructs made of a gel or foam based on spider silk protein.

The manufacture of a skin cream, on the basis of an innovative obtained composition with dissolved spider silk protein based on the above-described treated spider silk filaments, characterized to result in a protected, considerably stronger, softer skin.

### BRIEF DESCRIPTION OF THE DRAWINGS

Drawings have been added to clarify the invention. In these drawings, the density and thickness of the spider silk thread and the shape of the installations shown are purely for illustrative purposes only, unless otherwise stated.

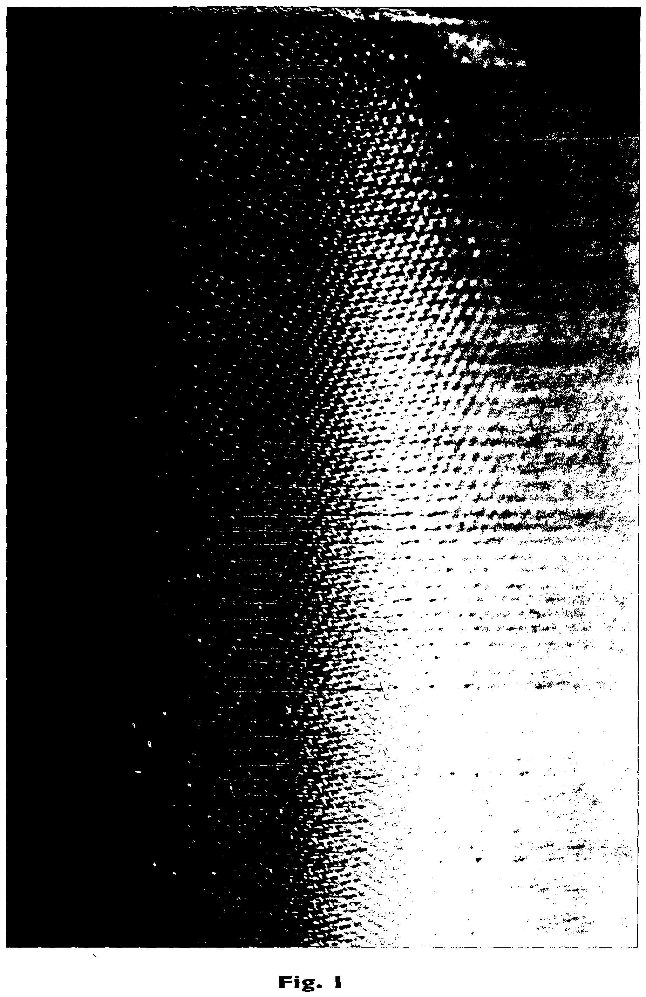

Fig. 1 Machine woven spider silk scaffold for a bioartificial tissue or skin tissue ie skin substitute. A complex textile fabric whose weft is completely encapsulating the warp, such that the warp is no longer visible. This creates a more three-dimensional structure compared to traditional woven textiles. The version shown here uses a high weave density.

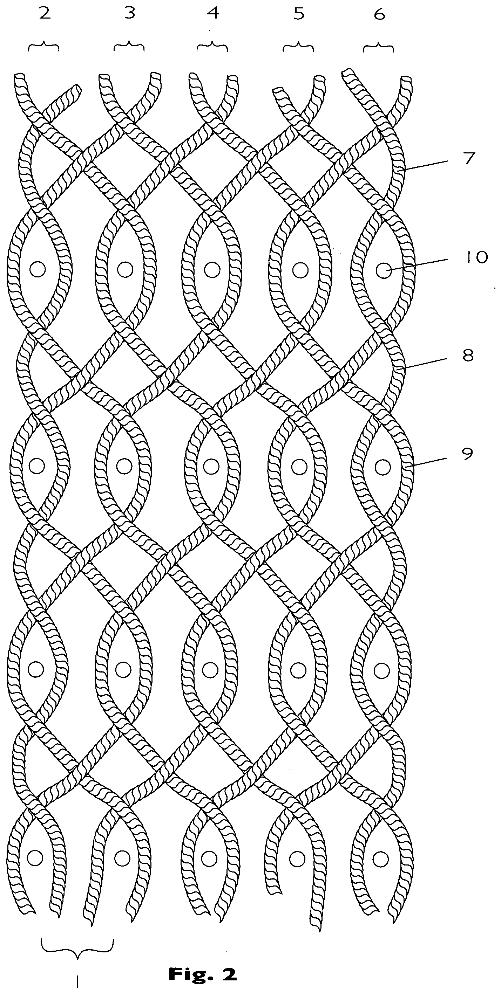

Fig. 2 A three-dimensional braiding technique. The illustrated variant is composed of five interwoven layers of spider silk thread 2,3,4,5,6, each separated by a distance 1 of 50 to 150 microns. Three spider silk threads 7,8,9 form the outer layer of the fabric. Multiple of these braided layers are attached to each other using warp yarns 10.

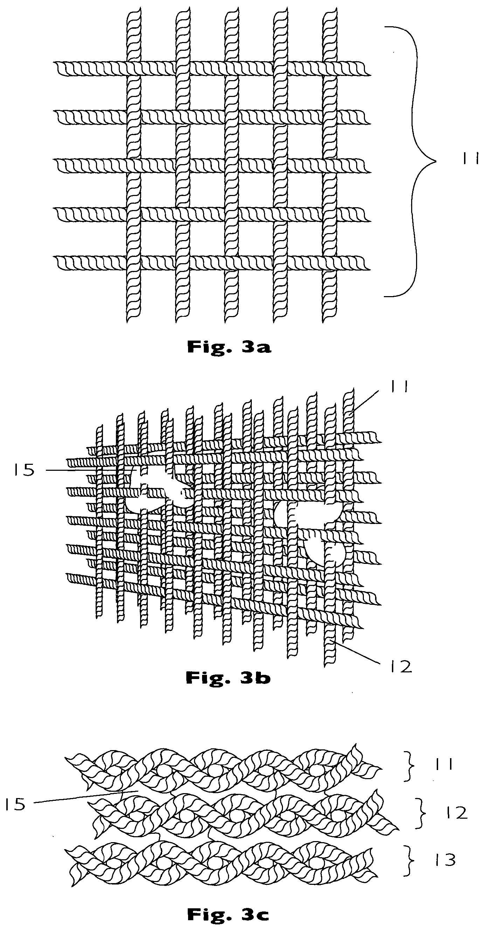

Fig. 3a - 3c A three-dimensional fabric made of multiple layers 11,12,13 of woven spider silk threads, attached to each other with a few drops of adhesive 15. Fig. 3a shows an isolated layer of the three-dimensional fabric, Fig. 3b shows the overlap of two layers combined with a limited amount of adhesive, and Fig. 3c shows a cross-section of the three-dimensional fabric composed of three layers including adhesives.

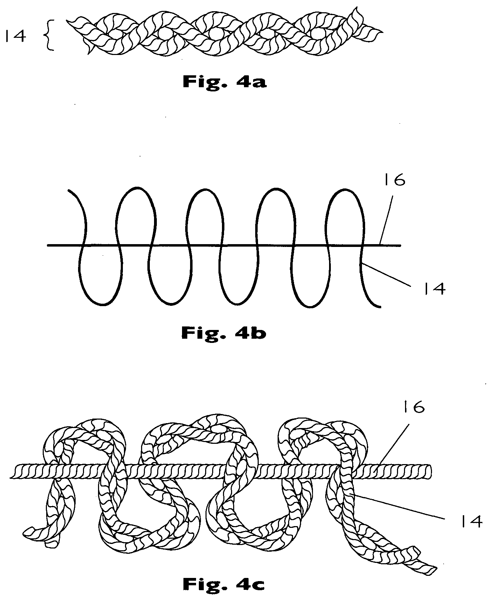

Fig. 4a - 4c A three-dimensional fabric formed from one (or more) woven spider silk threads 14. This fabric is folded (zigzag) in order to enlarge its total surface area, the fold is held in place with spider silk threads 16.

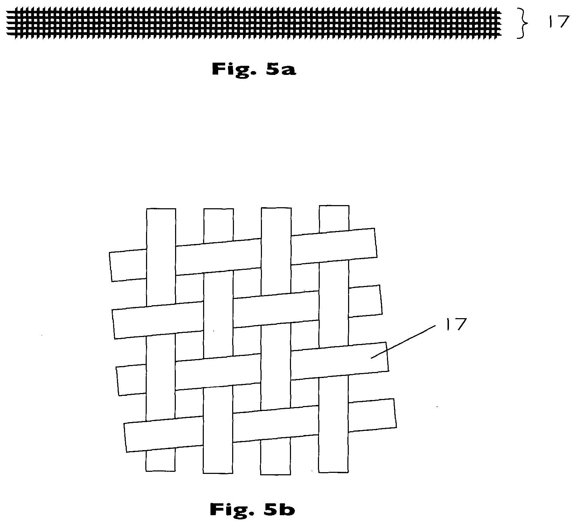

Fig. 5a - 5b A three-dimensional fabric consisting of braided narrow strips 17 made from spider silk thread. Fig. 5a shows a detail of Fig. 5b. This fabric can be combined with two or more layers as shown in Fig. 4.

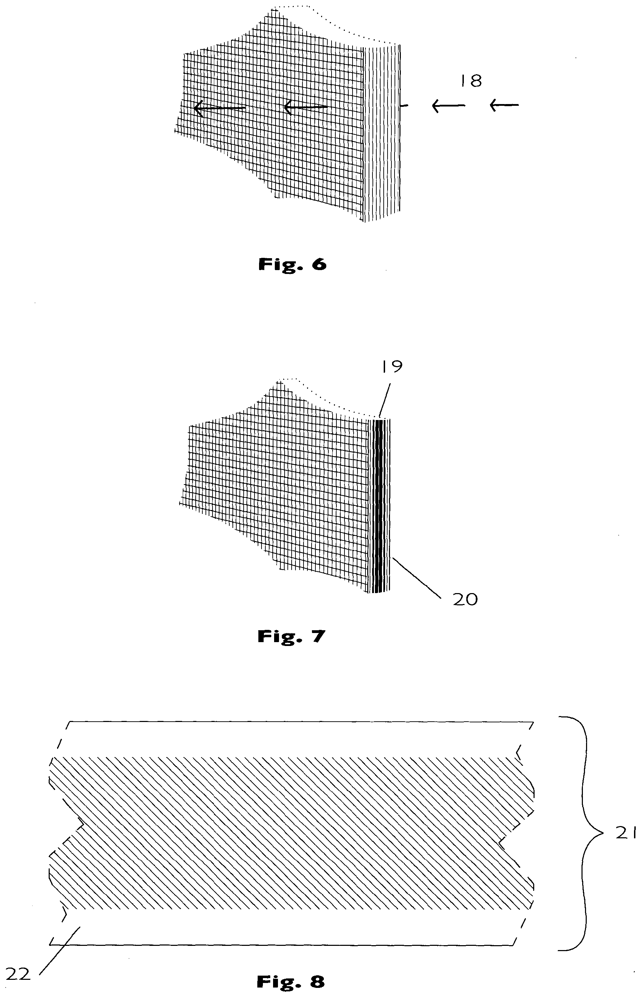

Fig. 6 Detailed view of the fine-meshed, enlarged total surface area of an industrial textile fabric according to the invention, applied to the surface of an apparatus aimed at heat transfer in combination with liquids and gases. Flow direction of the medium 18 is shown in the drawing.
Fig. 7 Detailed view of a fabric optimized for both heat exchange and thermal conductivity, the inner layers 19 relative to the outer layers 20 may consist of a compact three-dimensional fabric structure and have a higher weave density and atom density. The outer layers come into contact with more medium while the inner layers conduct the heat more efficiently over a larger distance.
Fig. 8 A cross-section of a thermal-conducting construct 21 coated with a thermalinsulating material 22.

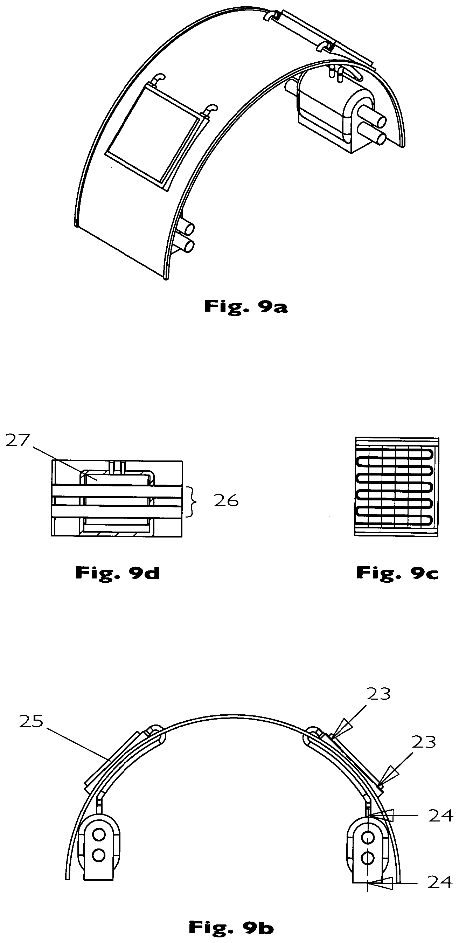

Fig. 9a - 9d These figures show a solar collector and heat exchanger of a solar water heater. The greenhouse of Fig. 9a shows a complete overview of the installation. Fig. 9b shows both the solar collector with its solar heat-absorbing surface 25, and the heat exchanger, both based on a spider silk fabric. A cross-section of the solar collector 23 can be seen in Fig. 9c. Fig. 9d is a cross-section of the heat exchanger 24 in a thermalinsulated housing. The heat exchanger consists of a number of cylindrical-shaped woven industrial textile fabrics 26 based on spider silk, which function as a heat exchanger between the solar-heated liquid medium 27 and the water that runs through the heat exchanger.

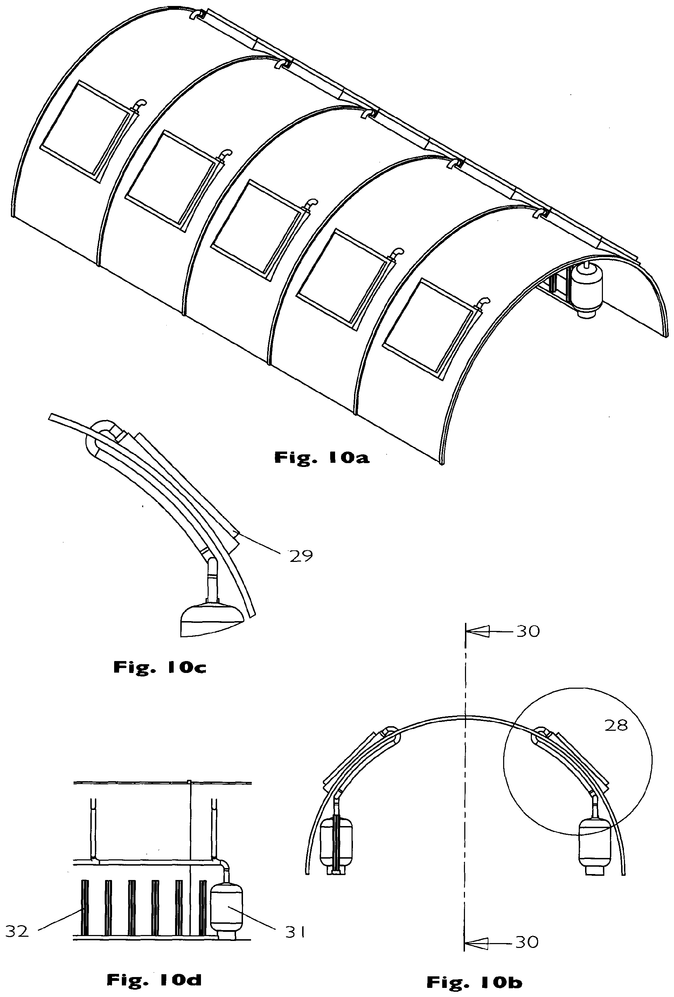

Fig. 10a - 10d A view of a solar collector used for terraforming using absorption cooling to extract water in areas with high humidity. The greenhouse in Fig. 10a gives a complete overview of the installation. Fig. 10b shows a cross-section of this installation with the absorption refrigeration and solar collector 28. Fig. 10c shows a detailed view of the solar collector, with its solar heat absorbing surface 29 based on an industrial spider silk textile fabric. Fig. 10d shows a cross-sectional view 30 of Fig. 10b wherein the housing 31 for the absorption cooling 31 and the cooling elements 32 are visible.

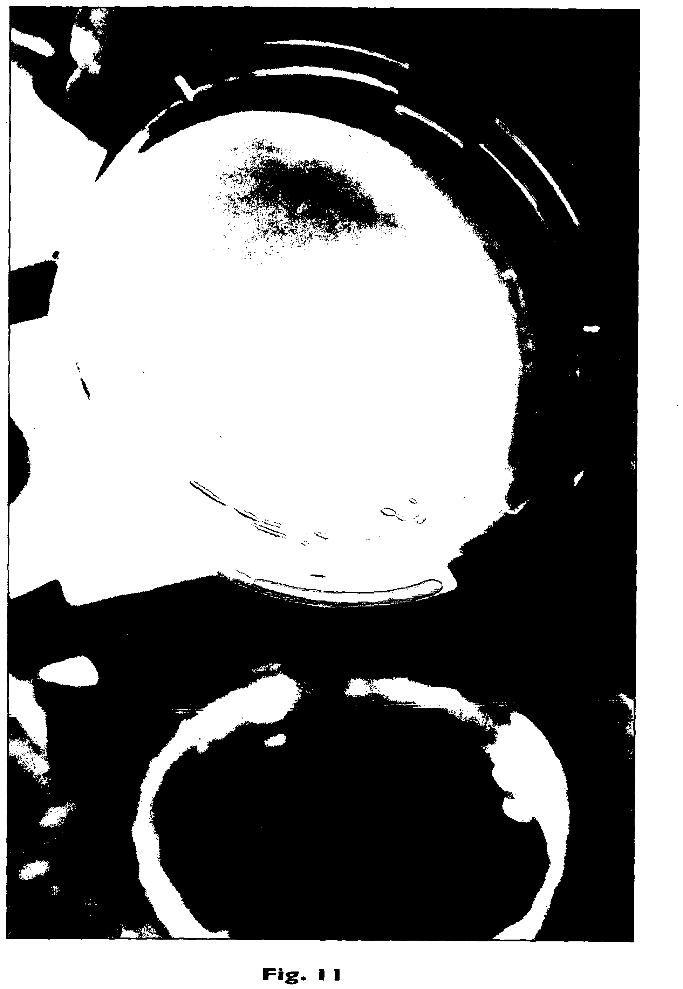

Fig. 11 Artificial tissue or skin tissue (artificial skin) based on the machine woven spider silk-scaffold from Fig. 1 embedded in a fully grown full-thickness skin model.

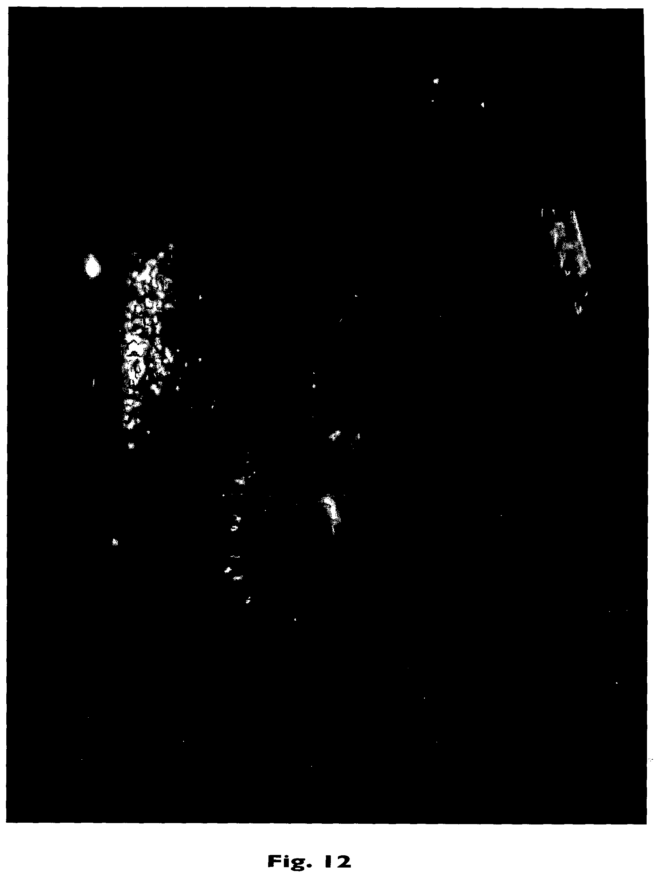

Fig. 12 Test at the Dutch Forensic Institute examining the increased resistance (bullet resistance) of the artificial skin (skin substitute) from Fig. 11 with respect to bullets and shrapnel.

### DETAILED DESCRIPTION OF THE PREFERRED EMBODIMENT(S)

The invention includes the manufacture of a treated spider silk filament, a spider silk thread and a textile fabric as well as support structure based on spider silk, which can be used for a broad number of applications, for example, a heat conductive textile fabric and / or support structure, a bio-artificial tissue and scaffold for a construct that hardens. The invention also comprises a composition with dissolved spider silk protein based on treated spider silk filaments, which can form a basis for an oil-in-water or water-in-oil-protective cream.

By inventively treating, twining, braiding or cabling one, two or more (recombinant) spider silk filaments derived from genetically modified organisms, or obtained through a wet or electro-spinning process of spider silk protein derived from genetically modified organisms, a spider silk thread, cable or braid is created which among other things is stronger than its alternatives. When describing this invention, we refer to the braided, cabled as well as the twined thread when we use the term spider silk thread, unless otherwise stated.

When using spider silk produced by the hybrid silkworm, the cocoon is first boiled, then it goes through a cooling process before the spider silk filament is degummed and reeled. Filament = continuous, endless thread. From Lewis it is known that this spider silk filament can be wound onto reels in order to be used for further processing. The spider silk filament has a thickness of 3-7 microns, this makes the filament unsuitable for use in standardized industrial weaving processes. After reeling the spider silk filament can be treated. To obtain optimal results this treatment process should take place right after the degumming process. The method of this process depends on the purpose and we distinguish four broad areas of applications: cosmetics, medical & biomedical, clothing & textiles and thermal/heat conduction.

In order to make the spider silk filament suitable for the manufacture of a spider silk thread that can be used for a textile fabric and / or support structure for the use in cosmetic, medical and biomedical applications (for example, a reinforced artificial skin) or as an ingredient for a composition based on spider silk protein (like protective skin creams), it is necessary to work with at least one component selected from the group consisting of, but not limited to: vitamins, hormones, antioxidants, chelating agents, antibiotics, preserving agents, fragrances, dyes, pigments, cell adhesion enhancers and other cosmetic, medical or dermatological active substances.

An example of this is camphor, a substance that can be used for the manufacture of spider silk bandages, plasters or plaster casts, based on its antipruritic and cooling effect, but also because of its inhibitory effects on inflammation and swellings. Spider silk filaments treated with camphor can also be an ingredient for skin cream because of its positive effect against acne and pleasant fragrance. For this purpose, the spider silk filament will be immersed in an oil or water bath with dissolved camphor 10 wt.% for 2 hours, after which further treatments follow depending on the application.

Another example is perfumes, with the help of the emulsifiers polysorbate 20 and polysorbate 80 essential oils and perfume oils can be mixed with water in order to treat spider silk filament. Mix the essential oil or perfume oil 15 wt.% with polysorbate 20 85 wt.% and add it to the water bath wherein the spider silk filament is treated.

An example of an antioxidant is Antirans 1 wt.%, which is a composition of citric acid, vitamin E, a fat-soluble vitamin C, glyceryl stearate, glyceryl oleate, and lycithin. Antioxidants have a wide application for use in cosmetic and biomedical applications, however their efficiency can reduce quickly, to counter this Disodium EDTA 1 wt.% should be added, this is a chelating agent and thus protects the antioxidant.

An example of a preservative which can be added to the above mentioned water bath for cosmetic applications is Biokons 0.3 to 2 wt.%, this is also a perfume and therefor does not have to be listed as preservative when describing the ingredients of the application. When used for biomedical applications, the substances discussed above, should (if possible) be applied as a coating or film, with a surface texture that guides cell alignment.

In order to manufacture a textile fabric and / or support structure for clothing and textile materials based on spider silk filaments, these filaments should be twined, cabled or braided into a spider silk thread. Prior to the twining, one needs to ensure that the proteins in the spider silk filament get an even better bonding with each other (based on contraction) by treating them with an potassium aluminum sulfate, KAl(SO₄)₂·12H₂O solution. In demineralized water an amount of 0.4 wt.% of potassium aluminum sulfate should be dissolved, depending on the amount of spider silk filament to be treated. The potassium aluminum sulfate solution is first brought to its boiling point and then cooled to a temperature of 21 degrees Celsius. The spider silk filament stays immersed in this solution for 24 hours. After the bath with potassium aluminum sulfate, the spider silk filament should be rinsed thoroughly with water of 21 degrees Celsius, and then passed through a physiological saline solution 0.9 wt.% NaCl and dried. This treatment enhances the spider silk filament and does not or hardly impact its flexibility.

In order to prepare the spider silk filament for the manufacture of a textile fabric and / or support structure with improved heat conductivity an alternative method is used. This treatment affects only the outer layer of the spider silk filament, in order to obtain a better thermal insulation. For this purpose, the spider silk filament is treated in a bath of citric acid 1.2 wt.% C₆H₈O₇ at a temperature of 21 degrees Celsius. Then, the spider silk filament should be rinsed thoroughly with a physiological saline solution, 0.9 wt.% NaCl, and dried at 21 degrees Celsius. A thread with improved thermal conductivity is also obtained by applying the above-described treatment with potassium aluminum sulfate. When these treatments are applied to spider silk filaments that are stretched up to 1.2 times their original length the treatments will have a maximal effect on the thermal conductivity, but this goes at the expense of flexibility.

After this treatment, the spider silk filament can be processed into a spider silk thread or yarn suitable for further processing, for use in for example a textile fabric. Two or more spider silk filaments are twined, cabled or braided, preferably three spider silk filaments of each 5-7 microns thick are combined into a spider silk thread of 15-20 microns. The linear density of the spider silk thread is selected to meet specific properties desired for the textile fabric and / or support structure, such as porosity and flexibility. The spider silk-thread may also be textured by heating the filament before the twine, braid or cable-process -after which it is cooled down again-, or by firmly twining the filaments - after which the thread is heated and partly un-twined-, or by other known processes to promote its softness, elasticity, thermal insulation, thermal conductivity and moisturewicking properties.

After obtaining of a spider silk thread, which can be used for industrial weaving processes, the thread itself can also be twisted, cabled or braided together to manufacture products like threads and cables.

By applying the above-described spider silk thread for the manufacture of a textile fabric and / or support structure, a material is obtained that is much stronger and more flexible than the present-day alternatives and that can be used as a cover / skin for designed objects, such as cars, airplanes, everyday objects and also people in the form of clothing.

By applying the above-described spider silk thread for the manufacture of a textile fabric and / or support structure, an innovative obtained enlarged total surface area is created which is suitable for use in heat exchange with a medium such as solids, liquids and gases, as well as for the application as a support structure for liquids which harden or organic materials which grow together.

A basic version of this invention can be based on any textile fabric, be it woven, knitted, braided, etc. Referring to the drawings, a woven construct is shown (Fig. 1 & 2), any known weave pattern can be used. The weave pattern used herein, includes a weft that is completely encapsulated by the warp, such that the warp is no longer visible and can even ben replaced with a different material, while still maintaining a surface that is entirely made of spider silk.

A complex three-dimensional support structure with a significantly enlarged total surface area can be obtained by applying three-dimensional weaving or braiding techniques, an example of a three-dimensional braiding technique (Fig. 2) consisting of at least two layers can be used as a scaffold for tissue engineering, thanks to its improved cell adhesion through its enlarged total surface area. The distance 1 between the layers 2,3,4,5,6 in Fig. 2 is 50 to 150 microns, allowing for cells to still reach their medium while not falling through. The depicted support structure consists of five layers, of which each is connected to at least one other layer. In this manner three spider silk threads 7,8,9 and the warp threads 10 form the outer layer. A complex three-dimensional support structure can also be obtained by the deformation and combination of layers based on the above-described basic textile fabrics. Combining layers 11,12,13,14 is possible using a flat (Fig. 3c) or wavy (Fig. 4b, 4c) structure, the layers can be adhered by means of spider silk protein in gel form 15 or collagen in case of biomedical applications, or stitched together with spider silk thread 16. A complex three-dimensional support structure (Fig. 5a, 5b) can also be obtained by braiding narrow strips based on the above-described basic textile fabrics 17.

When using the above-described textile fabrics for heat exchange, the density of the fabric can be adjust to optimally conduct heat for its application. (Fig. 6) With the same purpose a composite textile fabric with different densities can also be used. (Fig. 7) For this construct, the inner layers 19 relative to the outer layers 20, have a higher weave density and the spider silk filaments used have a higher atomic density. Because of this, this construct can optimally conduct heat in the inner layers and better exchange heat with a medium in the outer layers. The atomic density and thus the thermal conductivity of the textile fabric can be affected by treating it with citric acid, potassium aluminum sulfate, freon, or by boiling it in water and cooling it down. Resulting in a material that is less flexible, but with a higher atom density and enhanced thermal-conducting properties. It is also possible to increase the thermal-conducting properties of the textile fabric up to 1.2 times by stretching it to 1.2 times of its original length.

The surface 21 of a device with the aim of conduction heat can be insolated 22 in order to minimize heat loss. (Fig. 8) This can be done by means of existing techniques, but also by applying the above-described treatments on the construct itself instead on threads or filaments.

An optimum solar heat exchanger uses a spider silk textile fabric based on spider silk thread that has been colored black through genetic intervention or whose surface is painted matte-black. A matt-black spider silk flexible textile fabric is ideal for use as a solar collector 25 of a solar water heater (Fig. 9a - 9d), where in a heat-insulated housing a number of cylindrical-shaped spider silk textile fabrics 26 function as a heat exchanger between a solar-heated liquid medium 27 and the water that runs through the installation. A matt-black textile fabric is also ideal as a variation in a different form with the same function, or as a variation that transfers heat to air for heating of buildings, or as a solar collector wherein spider silk itself is the heat conducting medium, or as a solar collector 28 used for terraforming (Fig. 10a - 10d) on the basis of absorption cooling 31 and water extraction in areas with high humidity, working purely on solar heat. Ideal for environments where there is little water but high humidity, for example the Middle East.

The above-described textile fabric can also be applied as a scaffold for tissue engineering, a bio-artificial tissue, in this example (Fig. 11) in the form of an (artificial) skin. When weaving spider silk thread, two important features have to be taken into account regarding the shape of the scaffold in order for the scaffold to optimally fuse with the chosen skin model. The following embodiment is not according to the invention.
(a) The skin model used for the human skin equivalent should be exposed to air at the top and fed from the bottom. Because of this, a petri-dish is used with an permeable membrane that supports the skin model and its embedded scaffold. Partly because the seeded cells are fed from the bottom, the scaffold itself has to be permeable also with openings ranging from 50 to 150 micrometers. These openings must not be greater because of the size of the seeded cells.
(b) Increasing the total surface area of the scaffold ensures that more cells will adhere to the scaffold, and thus contributes to the strength of the final skin. Because the scaffold based on spider silk should be embedded in the skin model (artificial skin), the following five steps complement existing human skin equivalent protocols.
   1.) The scaffold based on fibers / thread is, if necessary, cleaned with sodium dodecyl sulfate and then rinsed with deionized water. The scaffold is then treated with plasma, because of the overlap of the fibers, in order to get it sterile. A scaffolds based on films / porous structures / sponges is heat-treated.
   2.) A fibroblast culture obtained according to the desired skin model is created and processed with collagen into a liquid gel; the ratio between the fibroblast-medium consisting of 20 wt.% fibroblasts and the collagen is 6:1.
   3.) This liquid collagen / fibroblast mixture is poured over the scaffold and stored away at 37.5 degrees Celsius and 5% CO₂ in a CO₂ incubator for 2 to 3 days. This solidifies / hardens and forms a dermis wherein the scaffold is integrated.
   4.) Subsequently a keratinocyte culture, obtained according to the desired skin model, is applied on top of the dermis.
   5.) After 1 to 2 days in the CO₂ incubator, the keratinocytes will be exposed to air for a duration of 14 days in order to grow and differentiate. During this period the medium should be replaced three times per week. The fibroblasts, together with the collagen and the scaffold, form an artificial dermis on which the keratinocytes grow. Because the model is cultivated under these conditions after a period of two weeks a lifelike (artificial) skin will form, a skin with the same layers normally present in real skin, reinforced by the spider silk textile scaffold.

The use of this scaffold for tissue engineering is not only limited to skin, but can also be used for other organs and tissues.

The above-described scaffold shows a better cell-adhesion when applied for tissue engineering, based on its material and the applied weave. Spider silk in itself already has the qualities for good cell-adhesion, but the increased total surface area of the scaffold and the above-described combination with collagen enhances these properties. In addition to enhance these properties even more, the spider silk filament can be treated not according to the invention with cell adhesion enhancers, for example: fibronectin, selectin, integrin and cadherin.

The above-described scaffold stimulates, through its material, weave and treatment, the growth and function of cells to an increased degree, and has significant influence on skin growth. Traditional silk has been used on a commercial scale for suture materials since the end of the 19th century, and has proven to be an effective biomaterial. Several studies show the growth rate and activity for a variety of cell types increases when combined with silk with reduced levels of sericin. By applying the above-described scaffold for tissue engineering a textile fabric and / or support structure is obtained that stimulates the growth and function of cells to an increased degree, and has significant influence on skin growth.

As a result of its material and weave, the above-described scaffold degrades in the human body with a controlled speed. A scaffold for human skin equivalents should preferably degrade at a similar rate as the growth of new tissue, in order for the new tissue to be integrated into the surrounding host tissue. This process is for silk and spider silk a result of proteolysis, of which proteases have the greatest impact. The speed at which spider silk degrades is very dependent on the type of spider silk used, thanks to its modular design the extent to which spider silk degrades can be manipulate via the GMO (genetically modified organism), but the rate at which spider silk degrades mainly depends on the shape of the scaffold. The above-described enlarged total surface area will degrade faster compared to a scaffold with a higher density and smaller total surface area. A tailor-made version can be obtained by manipulating the required weave density and total surface area.

The above-described scaffold shows, because of its material, weave, treatment and the speed at which it degrades in the body, a reduced pathological immune response. The main feature of spider silk when used in human skin equivalents is biocompatibility. Spider silk, unlike normal silk, does not have the immunogenic sericin coating, which is identified as the source of immunogenic reactions. By applying the above-described scaffold for tissue engineering, a textile fabric and / or supporting structure is obtained which will trigger a reduced pathological immune response, thanks to the use of spider silk. There will also be a reduced pathological immune response because the scaffold will degrade more quickly, based on its enlarged total surface area.

Through the application of the above-described scaffold for tissue engineering, a stronger human skin is obtained. This skin is ideal for skin grafting, for example, for patients with bedsores. When you scale up the thickness of the scaffold and combine it with tissue engineering, as happened for the bulletproof skin project, a construct is obtained with a number of new features, for example, enhanced tolerance for impacts from bullets and shrapnel. (Fig. 12) These principles not only apply to the combination with tissue engineering but also for all other materials that are first liquid and later harden, both synthetic and organic in nature. For example: rubber, cement, concrete, plastics, kombucha and other organic and synthetic hardeners.

Through the application of the above-described scaffold for tissue engineering, a skin is created which exhibits a prolonged growth and stimulates stem cell production. A normal in vitro skin grows on average five weeks and then begins to die because the production of stem cells stagnates / stops. Because of the use of the above-described scaffold for the bulletproof skin project, the skin continued to grow even after two months. It is unknown what exactly happened, except that the stem cell production did not stagnate. Further research is needed. There probably is an amino acid from the spider silk that allows for the stimulation of stem cell production, which makes the skin continue to grow. This embodiment is not according to the invention.

All of the above-described treatments and applications apply also for similar non-woven constructs made of a gel or foam based on spider silk protein, or produced by salt leaching to create porous constructs, or spongy or porous constructs produced by gas foaming, phase separation, or other known techniques. The treatments and applications also apply to similar products made of other materials for which spider silk protein can be processed as a film or coating. This embodiment is not according to the invention.

For the preparation of the above-described gel, foam, film or coating, spider silk protein can be used, obtained by known dissolving methods of spider silk filaments, which are obtained from genetically modified organisms and processed according to the present invention. This way a composite solution is obtained for cosmetic, medical and biomedical applications. This composition consists of spider silk protein and one or more of the above-described additives with which the spider silk filament was treated. Additives from the group consisting of, but not limited to: vitamins, hormones, antioxidants, chelating agents, antibiotics, preserving agents, fragrances, dyes, pigments, cell adhesion enhancers and other cosmetic, medical or dermatological active substances.

The manufacture of a skin cream, on the basis of the above-described innovative obtained composition with dissolved spider silk protein based on treated spider silk filaments, characterized to result in a protected, considerably stronger, softer skin. This embodiment is not according to the invention. For this purpose, the composition comprises a hydrophilic oil-in-water emulsion or a hydrophobic water-in-oil emulsion:
1.) an aqueous phase that makes up 50% to 98.9% by weight relative to the total weight of the composition, mixed with a broad-spectrum preservative 0.9 wt.% of phenoxyethanol and 0.1 wt.% ethylhexylglycerine.
2.) a fatty phase that makes up 0.1% to 50% by weight relative to the total weight of the composition, wherein up to 25% of the total weight is spider silk protein.
3.) at least one additive selected from the group consisting of, but not limited to: emulsifiers, or a pseudo-emulsifiers, such as spermaceti, cetyl alcohol, lecithin, lauryl-4, emulsan, potassium carbonate, Polysorbate 20, Polysorbate 80, Stearic Acid, Hydrogenated Palm Glycerides.
4.) an additive selected from the group consisting of, but not limited to: emollients, such as lanolin, vaseline, squalane, paraffin oil, paraffin wax, isopropyl myristate, cyclomethicone, cetyl alcohol or caprylic / capric triglyceride.
5.) an additive selected from the group consisting of, but not limited to: surfactants, such as cocamidopropyl betaine, decyl glucoside, Facetenside Hx, glycinetensid or Lamepon.

### EXAMPLES OF PRACTICAL APPLICATIONS

The following examples are given for illustration purposes only and are not according to the invention. The examples concern the use of spider silk protein derived from genetically modified organisms and more specifically the use of spider silk thread and recombinant spider silk thread from genetically modified silkworms for:

### A: LIFE SCIENCES

### EXAMPLE 1

Producing a stronger skin for plastic surgery: a human skin equivalent skin substitute based on spider silk thread can mean a significant advance, particularly for people with bedsores, war victims with large wounds, burn victims, etc.

### EXAMPLE 2

Weaving two-dimensional scaffolds for tissue engineering, which can be used for both in vitro and in vivo purposes.

### EXAMPLE 3

Weaving complex three-dimensional scaffolds, with different densities and textures, adjusted to a combination of one or more different cell types. These scaffolds may be used for in vitro research, and for the manufacture, repair and replacement of organs, blood vessels, artificial ligaments and tendons, etc.

### EXAMPLE 4

The use as a medium for three-dimensional printers for the production of scaffolds for tissue culture. The thread will be guided by the 3D printer and glued with a hardener like collagen, spider silk protein, or another alternative.

### EXAMPLE 5

Weave of the above-described scaffolds for tissue engineering aimed at improved or controlled a) promotion of cell adhesion, b) stimulation of cell growth, c) reduced pathological immune responses, d) adjusted biodegradability and e) stimulation of stem cell production.

### EXAMPLE 6

The manufacture of an inner lifting bra or corset, which is many times stronger than present-day alternatives by the use of spider silk.

### EXAMPLE 7

The use of spider silk protein, extracted from spider silk filament after being treated with camphor using the above-described method, for use in silk sprays aimed to treat open wounds and burns.

### EXAMPLE 8

Weaving spider silk patches / pressure bandage / plaster, treated with camphor according to the method discussed above. The heat-conducting property of spider silk and the treatment with camphor help reduce the heat originating from the healing process and to reduce itching.

### B: GENERAL USE TEXTILES

### EXAMPLE 9

Weaving lighter, stronger and / or more flexible textiles.

### EXAMPLE 10

Weaving a lighter, more flexible and / or stronger structure for use in clothing: shirts, sweaters, underwear, sleepwear, outerwear, lingerie, bra, shirts, blazers/jackets, dresses/skirts, trousers, ties, socks, tights, scarves, hats, corsets, wedding dresses, suspenders, baby clothes, overalls, leggings and also for use in footwear: shoes, sneakers, boots.

### EXAMPLE 11

Weaving the above-described clothing specifically aimed at providing protection against high voltage.

### EXAMPLE 12

Weaving the above-described clothing specifically aimed at civilian bulletproof clothes. In particular, shirts, jackets, tops, but also utensils such as lightweight ballistic briefcases. The woven structure for these objects can also be combined with a synthetic hardener, which imitates the reinforcing properties of the artificial skin grown using a spider silk scaffold according to the above-described method.

### EXAMPLE 13

Weaving a lighter, more flexible and / or stronger structure for use in consumer articles such as chairs, sofas, bags, jewelry, purses, belts, parasols, umbrellas, book covers, flags, silk top lace wigs, carpets, bedspreads, bed sheets, pillowcases, sleeping bags, curtains, tensioning cables, straps, safety belts and flexible cables.

### EXAMPLE 14

Weaving a lighter, more flexible and / or stronger structure for use in vehicles such as the outside / skin / cover for cars, trailers, buses, trains, airplanes and other vehicles.

### EXAMPLE 15

Weaving a strong and / or flexible support for tape based on nanotubes or other adhesive material.

### C: USE OF TEXTILE FOR HEAT CONDUCTION

### EXAMPLE 16

Weaving thermal conductive textiles. For example, for industrial use but also for use in electric blankets, pillows, slippers, curtains, seat cushions, insoles, inside of gloves and shoes.

### EXAMPLE 17

Weaving a textile fabric / artificial-skin / shell / surface / scaffold / heat exchanger based on solar energy: for solar boilers with solar collectors aimed at heating water; for solar air heating; for sunscreens that can serve as a solar collector; for compression cooling and absorption cooling on the basis of a solar collector that directly or indirectly heats the absorption refrigeration unit for use in air-conditioning; for a similar absorption cooling unit which extracts moisture from the air and turns this into ice and water for use in among others industrial, agricultural, automotive and space applications, ideal for environments where there is little water but high humidity such as the Middle East. If this application is being used to terraform using greenhouses in desert areas, it should be combined with smart glass that filters infrared light at temperatures above 29 degrees Celsius, enrichment of the soil with microorganisms (eg imported from countries with manure surpluses), and other well-known techniques.

### EXAMPLE 18

Weaving a heat exchanger for use in buildings, for example, flexible lightweight radiators for central heating and underfloor heating.

### EXAMPLE 19

Weaving heatsinks / heat exchangers for electronics with an enlarge total surface area aimed at optimal thermal conductivity for air or liquid, if necessary in combination with a fan or pump. Ideal for use in data centers / server parks.

### EXAMPLE 20

Weaving flexible heatsinks / heat exchanger for liquids and gases for larger applications in the agricultural-, aerospace-, automotive-, ship- and aircraft industries.

### EXAMPLE 21

Weaving heat exchangers for boilers or absorption cooling, like absorption refrigerators and air conditioners, with a heat source based on: solar; combustion heat, the exhaust of cars, the motor cars; the outside / skin of cars or other objects; the operational heat from data centers / server parks.

### D: GENERAL USE OF SPIDER SILK PROTEIN IN NON-WO VEN FORM

### EXAMPLE 22

All previous uses of spider silk, utilizing its superior heat conducting property (C) based on foam, gel, powder or film instead of a woven construct.

### EXAMPLE 23

All previous uses of spider silk, aimed at the general use of fabrics for clothing and textiles (B) based on foam, gel, powder or film instead of a woven construct.

### EXAMPLE 24

All previous uses of spider silk for biotechnological applications (A) based on foam, gel, powder or film instead of a woven construct.

### EXAMPLE 25

The use of spider silk protein, extracted from spider silk filament after being treated with camphor using the above-described method, for use in hair products, shampoo, lotions, skin creams, gel-based make-up, an oily gel, a compacted powder, a cast powder or a stick. This is an improvement with respect to the use of normal silk present in some of these products, since spider silk is hypoallergenic, biocompatible and can easily be processed while retaining its properties. This in contrast to normal silk, whose properties are often completely destroyed when used for cosmetic purposes. Interesting properties of spider silk for this use are the silky smooth texture and the protective, moisturizing properties for the skin.

### EXAMPLE 26

The use of spider silk filament, spider silk thread and spider silk protein as a green and biodegradable medium for 3d printers.

### EXAMPLE 27

The use of a textile fabric and / or support structure based on spider silk or a similar construct based on spider silk protein for the manufacture of bio-degradable cups, cutlery, pill containers, plastic bags, packaging material, and other disposable consumer items.

### EXAMPLE 28

The use of degummed spider silk filament, processed into spider silk thread, functioning as stronger and thinner dental floss.

### EXAMPLE 29

The use of spider silk filaments processed into a nail polish brush, dental brush, powder brush and other cosmetic brushes.

### EXAMPLE 30

The manufacture of a magnetic core based on spider silk thread, spider silk filaments or a gel or foam based on spider silk protein for electrical, electromechanical and magnetic devices such as electromagnets, transformers, electric motors and inductors. This can take various shapes including; a straight cylindrical or square rod, found in car ignition coils or a C, U, E or E + I core and other known alternatives.

### EXAMPLE 31

The manufacture of a strong filament / thread, used for facelifts. With the use of narrow needles the filament is placed perpendicular to the lines of the wrinkles of the head and neck.

## Claims

1. A method for manufacturing a spider silk thread on basis of a spider silk filament in the manufacture of cosmetic, medical, textile or industrial applications, comprising the steps of:
(A) providing spider silk filament derived from genetically modified organisms;
(B) treating of the spider silk filament with at least one component selected from the group consisting of vitamins, hormones, antioxidants, chelating agents, antibiotics, preserving agents, fragrances, dyes, pigments, magnetic nanoparticles, nanocrystals, cell adhesion enhancers, thermal insulators, shrinkage agents and cosmetic, medical or dermatological active substances;
**characterised, in that** for obtaining a better bonding of the proteins in the spider silk filament the spider silk filament is treated with a 0.4 wt.% potassium aluminum sulfate KAl(SO₄)₂·12H₂O solution, wherein the treatment comprises the following subsequent steps:
bringing the potassium aluminum sulfate solution to its boiling point,
cooling the potassium aluminum sulfate solution to a temperature of 21 °C,
immersing the spider silk filament in the potassium aluminum sulfate solution for 24 hours,
rinsing the spider silk filament thoroughly with water of 21 °C,
passing the spider silk filament through a physiological saline solution 0.9 wt.% NaCl,
drying the spider silk filament;
twining, cabling or braiding of two or more spider silk filaments into the spider silk thread.

2. The method of claim 1, wherein the spider silk filament is obtained by a wet- or electrospinning process of spider silk protein derived from genetically modified organisms.

3. The method according to any one or more of claims 1-2, wherein three spider silk filaments of each 5-7 microns thick are combined into a spider silk thread of 15-20 microns.

4. Spider silk thread obtained according to a method according to any one or more of claims 1-3, wherein the spider silk thread is textured.

5. A method for the manufacture of a cosmetic, medical or industrial textile fabric and/or support structure, based on a spider silk thread obtained according to a method according to any one or more of claims 1-3 or a spider silk thread according to claim 4, comprising the steps of:
mechanically two-or three-dimensional weaving, braiding and / or knitting of the spider silk thread to form a stronger, more flexible, enlarged total surface area, which is suitable for use in heat exchange with a medium such as solids, liquids and gases, as well as for the application as a support structure for liquids which harden, as well as for general use in strengthening surfaces.

6. Textile fabric and / or support structure obtained by the method according to claim 5, wherein the total surface area obtained is tuned in such a way that the increased thermal conductivity can be optimally utilized for its application.

7. Textile fabric and / or support structure according to claim 6, wherein the textile fabric is dyed matt black for optimally absorbing solar heat.

8. Textile fabric and / or support structure according to any one of claims 6-7 wherein the obtained fabric is partly thermally insulated to reduce heat loss to a minimum.

9. The use of a textile fabric and / or support structure according to claim 7 in a solar collector for solar water heaters, solar air heating, compression refrigeration, absorption cooling and water extraction on the basis of these techniques.

10. A method for the manufacture of a bio-artificial tissue or skin tissue, comprising the steps of:
(A) providing textile fabric and /or support structure obtained by the method according to claim 5;
(B) treating of the textile fabric and / or support structure with at least one component selected from the group consisting of fibroblasts, keratinocytes and other cells and tissues.

## Patentansprüche

1. Verfahren zum Herstellen eines Spinnenseidenfadens auf Basis eines Spinnenseidenfilaments bei der Herstellung kosmetischer, medizinischer, Textil- oder industrieller Anwendungen, umfassend die folgenden Schritte:
(A) Bereitstellen von Spinnenseidenfilament, das von genetisch modifizierten Organismen abgeleitet ist;
(B) Behandeln des Spinnenseidenfilaments mit mindestens einer Komponente, die aus der Gruppe bestehend aus Vitaminen, Hormonen, Antioxidanzien, Chelatbildnern, Antibiotika, Konservierungsstoffen, Duftstoffen, Farbstoffen, Pigmenten, magnetischen Nanopartikeln, Nanokristallen, Zelladhäsionsverstärkern, Wärmeisolatoren, Schrumpfungsmitteln und kosmetisch, medizinisch oder dermatologisch aktiven Substanzen ausgewählt ist;
**dadurch gekennzeichnet, dass** zum Erhalten einer besseren Bindung der Proteine in dem Spinnenseidenfilament das Spinnenseidenfilament mit einer 0,4-prozentigen (Gew.-%) Lösung von Kaliumaluminiumsulfat KAl(SO₄)₂·12H₂O behandelt wird, wobei die Behandlung die folgenden aufeinanderfolgenden Schritte umfasst:
Bringen der Kaliumaluminiumsulfatlösung auf ihren Siedepunkt,
Abkühlen der Kaliumaluminiumsulfatlösung auf eine Temperatur von 21 °C,
Eintauchen des Spinnenseidenfilaments in die Kaliumaluminiumsulfatlösung für 24 Stunden,
gründliches Spülen des Spinnenseidenfilaments mit Wasser mit 21 °C,
Hindurchlassen des Spinnenseidenfilaments durch eine physiologische Kochsalzlösung 0,9 Gew.-% NaCl,
Trocknen des Spinnenseidenfilaments;
Verwinden, Verdrillen oder Flechten von zwei oder mehr Spinnenseidenfilamenten zu dem Spinnenseidenfaden.

2. Verfahren nach Anspruch 1, wobei das Spinnenseidenfilament durch einen Nass- oder Elektrospinnprozess von Spinnenseidenprotein erhalten wird, das von genetisch modifizierten Organismen abgeleitet ist.

3. Verfahren nach einem der Ansprüche 1-2, wobei drei Spinnenseidenfilamente, die jeweils eine Dicke von 5-7 Mikrometern aufweisen, zu einem Spinnenseidenfaden von 15-20 Mikrometern kombiniert werden.

4. Spinnenseidenfaden, erhalten nach einem Verfahren nach einem der Ansprüche 1-3, wobei der Spinnenseidenfaden strukturiert ist.

5. Verfahren zum Herstellen einer kosmetischen, medizinischen oder industriellen Textilfaser und/oder Trägerstruktur, basierend auf einem nach einem Verfahren nach einem der Ansprüche 1-3 erhaltenen Spinnenseidenfaden oder einem Spinnenseidenfaden nach Anspruch 4, umfassend die folgenden Schritte:
mechanisches zwei- oder dreidimensionales Weben, Flechten und/oder Stricken des Spinnenseidenfadens, um eine stärkere, flexiblere und vergrößerte Gesamtoberfläche zu bilden, die zur Verwendung im Wärmeaustausch mit einem Medium wie Feststoffen, Flüssigkeiten und Gasen sowie zur Anwendung als Trägerstruktur für Flüssigkeiten, die aushärten, sowie zur allgemeinen Verwendung in Verstärkungsoberflächen geeignet ist.

6. Textilfaser und/oder Trägerstruktur, erhalten durch das Verfahren nach Anspruch 5, wobei die erhaltene Gesamtoberfläche derart abgestimmt wird, dass die erhöhte Wärmeleitfähigkeit optimal für diese Anwendung genutzt werden kann.

7. Textilfaser und/oder Trägerstruktur nach Anspruch 6, wobei die Textilfaser zur optimalen Absorption von Sonnenwärme mattschwarz gefärbt ist.

8. Textilfaser und/oder Trägerstruktur nach einem der Ansprüche 6-7, wobei der erhaltene Stoff teilweise wärmeisoliert ist, um den Wärmeverlust auf ein Minimum zu reduzieren.

9. Verwendung einer Textilfaser und/oder Trägerstruktur nach Anspruch 7 in einem Solarkollektor für Solarwarmwasserbereiter, Solarluftheizung, Kompressionskühlung, Absorptionskühlung und Wasserextraktion auf Basis dieser Techniken.

10. Verfahren zum Herstellen eines biologisch-künstlichen Gewebes oder Hautgewebes, umfassend die folgenden Schritte:
(A) Bereitstellen von Textilfaser und/oder Trägerstruktur, die durch das Verfahren nach Anspruch 5 erhalten wurde;
(B) Behandeln der Textilfaser und/oder Trägerstruktur mit mindestens einer Komponente, die ausgewählt ist aus der Gruppe bestehend aus Fibroblasten, Keratinozyten und anderen Zellen und Geweben.

## Revendications

1. Procédé de fabrication d'un fil de soie d'araignée sur la base d'un filament de soie d'araignée pour la fabrication de produits cosmétiques, médicaux, textiles ou des applications industrielles, comprenant les étapes de :
(A) fourniture d'un filament de soie d'araignée dérivé d'organismes génétiquement modifiés ;
(B) traitement du filament de soie d'araignée avec au moins un composant choisi dans le groupe constitué de vitamines, d'hormones, d'antioxydants, d'agents chélatants, d'antibiotiques, d'agents conservateurs, de fragrances, de colorants, de pigments, de nanoparticules magnétiques, de nanocristaux, d'activateurs d'adhérence cellulaire, d'isolants thermiques, d'agents de retrait et de substances actives cosmétiques, médicales ou dermatologiques ;
**caractérisé en ce que,** pour obtenir une meilleure liaison des protéines dans le filament de soie d'araignée, le filament de soie d'araignée est traité avec une solution de sulfate de potassium et d'aluminium KAl(SO₄)₂·12H₂O à 0,4% en poids, dans lequel le traitement comprend les étapes ultérieures suivantes de :
amenée de la solution de sulfate de potassium et d'aluminium à son point d'ébullition ;
refroidissement de la solution de sulfate de potassium et d'aluminium à une température de 21°C ;
immersion du filament de soie d'araignée dans la solution de sulfate de potassium et d'aluminium pendant 24 heures ;
rinçage abondant du filament de soie d'araignée avec de l'eau à 21°C ;
passage du filament de soie d'araignée dans une solution saline physiologique à 0,9% en poids de NaCl ;
séchage du filament de soie d'araignée ;
entortillage, câblage ou tressage de deux filaments de soie d'araignée ou plus pour obtenir le fil de soie d'araignée.

2. Procédé de la revendication 1, dans lequel le filament de soie d'araignée est obtenu par un procédé de filage humide ou électrostatique de protéine de soie d'araignée dérivée d'organismes génétiquement modifiés.

3. Procédé selon l'une quelconque ou plusieurs des revendications 1 et 2, dans lequel trois filaments de soie d'araignée de 5 à 7 microns d'épaisseur chacun sont combinés pour obtenir un fil de soie d'araignée de 15 à 20 microns.

4. Fil de soie d'araignée obtenu selon un procédé selon l'une quelconque ou plusieurs des revendications 1 à 3, dans lequel le fil de soie d'araignée est texturé.

5. Procédé de fabrication d'une étoffe textile et/ou d'une structure de support à usage cosmétique, médical ou industriel, sur la base d'un fil de soie d'araignée obtenu selon un procédé selon l'une quelconque ou plusieurs des revendications 1 à 3 ou d'un fil de soie d'araignée selon la revendication 4, comprenant les étapes de :
tissage, tressage et/ou tricotage mécanique(s) bidimensionnel(s) ou tridimensionnel(s) du fil de soie d'araignée afin de former une surface totale plus résistante, plus flexible et plus grande, qui est appropriée pour une utilisation dans l'échange thermique avec des milieux tels que des solides, des liquides et des gaz, pour l'application comme structure de support pour des liquides qui durcissent, ainsi que pour une utilisation générale dans le renforcement des surfaces.

6. Étoffe textile et/ou structure de support obtenue(s) par le procédé selon la revendication 5,
dans laquelle/lesquelles la surface totale obtenue est réglée de manière à ce que la conductivité thermique accrue puisse être utilisée de façon optimale pour son application.

7. Étoffe textile et/ou structure de support selon la revendication 6, dans laquelle/lesquelles l'étoffe textile est teinte en noir mat pour absorber de manière optimale la chaleur solaire.

8. Étoffe textile et/ou structure de support selon l'une quelconque des revendications 6 et 7, dans laquelle/lesquelles l'étoffe obtenue est partiellement isolée thermiquement pour réduire la perte de chaleur au minimum.

9. Utilisation d'une étoffe textile et/ou d'une structure de support selon la revendication 7 dans un collecteur solaire pour les chauffe-eau solaires, le chauffage solaire de l'air, la réfrigération par compression, le refroidissement par absorption et l'extraction d'eau sur la base de ces techniques.

10. Procédé de fabrication d'un tissu bio-artificiel ou d'un tissu cutané, comprenant les étapes de :
(A) fourniture d'une étoffe textile et/ou d'une structure de support obtenue(s) par le procédé selon la revendication 5 ;
(B) traitement de l'étoffe textile et/ou de la structure de support avec au moins un composant choisi dans le groupe constitué de fibroblastes, de kératinocytes et d'autres cellules et tissus.
